# EUROPEAN PATENT SPECIFICATION

(11) **EP 3 171 868 B1**
(45) Date of publication and mention of the grant of the patent: **30.09.2020**
(21) Application number: 14897969.3
(22) Date of filing: 25.07.2014
(51) Int. Cl.: A61K 31/167, A61J 1/00, A61K 9/08, A61K 47/02, A61K 47/04, A61K 47/10, A61K 47/20, A61P 25/04

(54) **PACKAGED ACETAMINOPHEN INJECTION SOLUTION PREPARATION**
KOMPRIMIERTES ACETAMINOPHENINJEKTIONSLÖSUNGSPRÄPARAT
PRÉPARATION DE SOLUTION POUR INJECTION D'ACÉTAMINOPHÈNE CONDITIONNÉE

(43) Date of publication of application: 31.05.2017
(73) Proprietor: Terumo Kabushiki Kaisha, Tokyo 151-0072 (JP)
(72) Inventor: SHIMODA, Masami, Kanagawa 259-0151 (JP); YAGO, Seiji, Kanagawa 259-0151 (JP); SAKAGUCHI, Chiharu, Kanagawa 259-0151 (JP); IWAKIRI, Chisato, Kanagawa 259-0151 (JP)
(74) Representative: Casalonga
(86) International application number: PCT/JP2014/003949
(87) International publication number: WO 2016/013049

(56) References cited:
- WO-A1-2010/121762
- FR-A1- 2 926 291
- JP-A- 2006 187 429
- JP-A- 2006 217 975
- JP-A- 2006 217 975
- JP-A- 2009 543 851
- JP-A- 2010 081 971
- JP-A- 2011 503 198
- JP-A- 2012 188 397
- JP-A- 2012 524 738
- JP-A- 2013 523 864
- JP-A- 2014 505 081

## Description

### Technical Field

The present invention relates to a packaged acetaminophen injection solution preparation. More particularly, the present invention relates to a packaged acetaminophen injection solution preparation which excels in storage stability, permitting acetaminophen to remain stable in the solution even after storage at room temperature for a long period of time.

### Background Art

Acetaminophen (synonymous with paracetamol) is a mild antipyretic/analgesic which is free of stomach irritation, Inhibiting blood coagulation, kidney disorder, and such side effects as excitement and sleepiness. Moreover, it involves no problems with drug dependence and abstinence symptoms. This is the reason why acetaminophen in the form of oral drug and suppository is one of the most popular antipyretic/analgesic being used throughout the world.

Acetaminophen is also available in the form of injection solution preparation (which is an aqueous solution of acetaminophen filled in a container). Since its appearance on the overseas market in 2002, it has been approved for production and sale in about 80 countries (except for Japan) including United States and European counties. It is now being used for the standard treatment of postoperative pain.

Among known acetaminophen injection solution preparations is one which is an aqueous solution of acetaminophen filled and sealed in a glass bottle in a nitrogen gas atmosphere after deoxygenation (by inert gas bubbling) for stability during long-term storage and subsequent moist heat sterilization (See USP6028222, hereinafter referred to as Patent Document 1). There has been proposed a method for keeping the oxygen content in the aqueous solution of acetaminophen as low as 0.4 to 4 mg/L by subjecting the aqueous solution to ultrasonic action and either or both of suction and microbubbling (See US2009044700A, hereinafter referred to as Patent Document 2). Another aqueous composition comprising paracetamol is described in WO 2010/121762. It is in the form of an infusion solution and has an electrical conductivity of at most 200 µS.cm⁻¹. Said composition does not contain any C₁-C₆-alkanols or any polyethylene glycol.

Following the technology described in USP6028222 and US2009044700A mentioned above, the present inventors prepared an acetaminophen injection solution preparation filled in a bag by subjecting an acetaminophen aqueous solution to deoxygenation (by inert gas bubbling), filling and sealing it in a container (specifically, a bag of organic polymer sheet) in an inert gas atmosphere, and moist heat sterilization (pressurized with an inert gas such as nitrogen). As the result of storage stability test, the acetaminophen injection solution preparation was found to be unsatisfactory in long-term storage stability despite the deoxygenation treatment in each step.

It is an object of the present invention to provide a packaged acetaminophen injection solution preparation as defined in claim 1, which is so superior in long-term storage stability that it retains acetaminophen in an extremely high ratio even after storage for three years or longer at room temperature and hence it can be stored safely over a long period of time.

The object of the present invention is to provide a packaged acetaminophen injection solution preparation which is held in a container, specially a baglike container, formed from organic polymer film or sheet, the container being suitable for long-term safe storage at room temperature and being light in weight and good in handleability without breakage.

In order to achieve the foregoing objects, the present inventors carried out extensive researches, which suggested that the long-term storage stability is not attributable to the oxygen control involved in the production of the acetaminophen injection solution preparation. In order to clear whether or not this is true, the present inventors produced samples of acetaminophen injection solution preparations in different ways as follows and tested them for storage stability.

That is, the present inventors produced the acetaminophen injection solution preparation in the above-mentioned manner including the step for preparing an acetaminophen aqueous solution before filling in a container, the step for filling the acetaminophen aqueous solution in the container, the step for moist heat sterilization (in other words, high-pressure steam sterilization and autoclaving) on the container filled with the acetaminophen aqueous solution; all the steps being performed with the introduction of an inert gas or in an inert gas atmosphere. In addition thereto, the present inventors produced the acetaminophen injection solution preparation in a manner in which one or two or all of the steps are performed in air (in place of inert gas atmosphere).

The results of the foregoing experiments indicate that, unlike the known data described in Patent Documents 1 and 2 mentioned above, the long-term storage stability of the acetaminophen injection solution preparation is affected only little by the exposure to oxygen which occurs in the step for preparing the acetaminophen aqueous solution before filling in the container, the step for filling the acetaminophen aqueous solution in the container, and the step for moist heat sterilization applied to the container filled with the acetaminophen aqueous solution.

The present inventors conjectured from the foregoing that the deterioration and decomposition of the acetaminophen injection solution preparation are little affected by the oxygen control carried out in its manufacturing stage in a short period of time but they occur during storage after production. As a result, they reasoned that acetaminophen does not react with oxygen rapidly but rather slowly.

The present inventors have presumed as follows.
The oxygen control at the time of preparation of acetaminophen aqueous solution (by inert bas bubbling such as nitrogen gas), the oxygen control at the time of filling of acetaminophen aqueous solution into a container (in an inert gas atmosphere), and the oxygen control at the time of moist heat sterilization (pressurized with an inert gas) are not necessarily important. To remove oxygen in a packaged salable acetaminophen preparation at any gradually is important. It would be possible to obtain an acetaminophen injection solution preparation superior in long-term storage stability if acetaminophen in its aqueous solution is protected from deterioration and decomposition during storage by removing oxygen in the packaged salable acetaminophen preparation. With the foregoing in mind, the present inventors reviewed various possible means.

Finally, the present inventors found that the foregoing object is achieved by filling an acetaminophen aqueous solution in a double container form which is composed of an oxygen-permeable inner container and an oxygen-impermeable outer container, with the space between two containers being stored with an oxygen scavenger. During storage, the double-layered container permits oxygen to be removed gradually from the acetaminophen aqueous solution and the head space of the inner container holding the acetaminophen aqueous solution. The considerable oxygen reduction from the inner container achieved in this manner contributes to the long-term storage stability of the acetaminophen injection solution preparation. This finding led to the present invention.

### Summary of Invention

The means for achieving the object is as described below.

A packaged acetaminophen injection solution preparation comprising:
an oxygen-impermeable packaging container, and
an acetaminophen injection solution preparation including a sealed oxygen-permeable container and an acetaminophen aqueous solution of pH 4 to 8 containing acetaminophen as an active ingredient filled in said sealed oxygen-permeable container, and
an oxygen scavenger.
wherein:
   - said acetaminophen injection solution preparation is a moist heat-sterilized acetaminophen injection solution preparation,
   - said oxygen-impermeable packaging container encloses said moist heat-sterilized acetaminophen injection solution preparation and said oxygen scavenger, and is sealed,
   - said oxygen-permeable container has an oxygen permeability not lower than 500 cm³/m²/day measured at 23 degree C and 90% RH under normal pressure (1013 hPa),
      and
   - said oxygen scavenger is positioned in a space between said sealed oxygen-impermeable container and said moist heat-sterilized acetaminophen injection solution preparation to absorb oxygen within said sealed oxygen-impermeable packaging container.

### Brief Description of Drawings

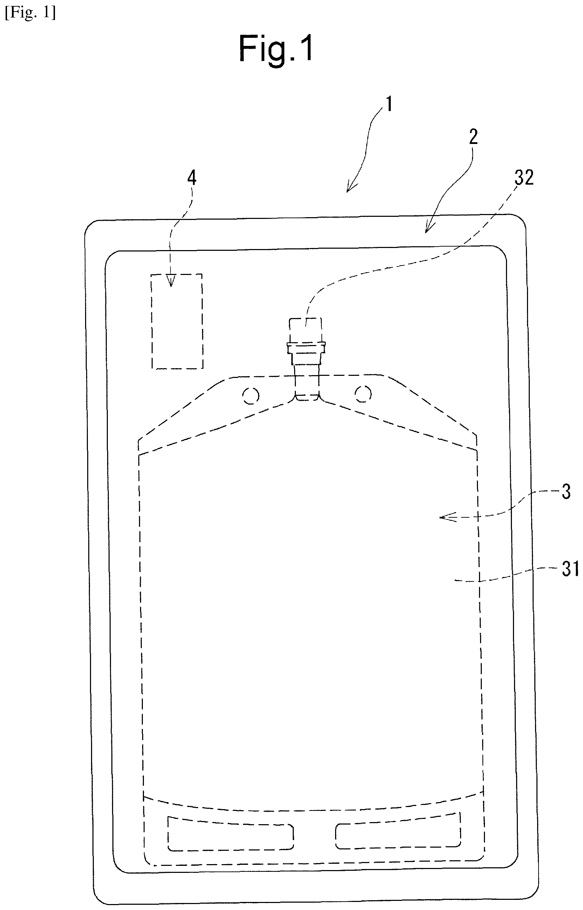

[fig. 1] Fig. 1 is a plan view of an embodiment of an acetaminophen injection solution preparation of the present invention

### Description of Embodiments

The following is a detailed description of the present invention.
A packaged acetaminophen injection solution preparation of this invention comprises an acetaminophen injection solution preparation and an oxygen-impermeable packaging container. The acetaminophen injection solution preparation is sterilized by moist heat sterilization (in other words, high-pressure steam sterilization or autoclaving). The oxygen-impermeable packaging container encloses the acetaminophen injection solution preparation and is sealed. The packaged acetaminophen injection solution preparation has an oxygen scavenger to absorb oxygen within the sealed oxygen-impermeable packaging container.

In other words, the present invention relates to an acetaminophen injection solution preparation which is composed of an acetaminophen aqueous solution containing acetaminophen as an active ingredient, an oxygen-permeable inner container holding it therein, which has undergone moist heat sterilization after filling, and a tightly sealed oxygen-impermeable outer container (in other words, packing container) holding together the inner container and an oxygen scavenger placed between the inner and outer containers.

The acetaminophen injection solution preparation comprises the sealed oxygen-permeable container and the acetaminophen aqueous solution filled in the sealed oxygen-permeable container. In particular, in the embodiment shown in Fig. 1, the packaged acetaminophen injection solution preparation includes the acetaminophen injection solution preparation, the oxygen-impermeable packaging container and the oxygen scavenger. The oxygen-impermeable packaging container encloses the acetaminophen injection solution preparation and the oxygen scavenger. Further, the oxygen scavenger is positioned in a space between the packaging container and the sealed oxygen-permeable container filled with said acetaminophen aqueous solution. But, the oxygen-impermeable packaging container or the sealed oxygen-permeable container may have the oxygen scavenger. The acetaminophen may be that of any type ordinarily used for injection solution, oral drug, and suppository. It may also be used in the form of physiologically acceptable salt.

The acetaminophen injection solution preparation of this invention includes the acetaminophen aqueous solution filled in the oxygen-permeable container has pH 4 to 8, preferably pH 5 to 6. With a pH value lower than 4, the acetaminophen aqueous solution is poor in storage stability; with a pH value higher than 8, the acetaminophen aqueous solution tends to stimulate the blood vessel (causing pain and inflammation to the blood vessel) when administered by injection.

The acetaminophen aqueous solution is capable of pH adjustment with any conventional pH adjustor for injection solutions. Examples of the pH adjustor include bases such as sodium hydroxide and potassium hydroxide, inorganic acids such as hydrochloric acid, and organic acids such as citric acid, lactic acid, acetic acid, succinic acid, and malic acid. It is desirable that the salts formed pH adjustment to be completed is less. Thus, as the pH adjustment, sodium hydroxide (strong base) and hydrochloric acid (strong acid) are preferred.

The acetaminophen aqueous solution to be filled in the oxygen-permeable container should be prepared from pharmacologically and physiologically acceptable water, such as distilled water, water, purified water, sterile purified water, water for injection, and distilled water for injection. These kinds of water are defined in Japanese Pharmacopoeia, 16th edition.
The acetaminophen aqueous solution should contain acetaminophen in an amount of 2 to 50 mg/mL, preferably 5 to 10 mg/mL, from the standpoint of solubility and the effectiveness and dose to be intended when used as an intravenous injection solution.

With an excessively low concentration, the acetaminophen aqueous solution needs to be administered in a large amount to the patient. With an excessively high concentration, the acetaminophen aqueous solution tends to cause precipitation at low temperatures on account of the low solubility of acetaminophen.
The acetaminophen aqueous solution should preferably contain, in addition to the foregoing pH adjustor, one or two or more species of tonicity agent, antioxidant, and buffer agent.

The tonicity agent may be selected from any existing ones used for injection solution. It includes, for example, nonionic ones, such as mannitol, sorbitol, inositol, glucose, propylene glycol, and glycerol, and ionic ones, such as sodium chloride. One or two or more of them may be used. Among these examples, mannitol is preferable from the standpoint of safety and stability to sterilization.
The tonicity agent should be added in an amount enough to make the acetaminophen injection solution have approximately or exactly the same osmotic pressure as serum (such that the ratio of osmotic pressures is from 0.9 to 1.1).

The antioxidant may be selected from any existing ones used for injection solution. It includes, for example, an organic compound having a thiol functional group (such as cysteine), the form of salt thereof and hydrate thereof (e.g., cysteine hydrochloride hydrate), a sulfite, such as sodium hydrogen sulfite, ascorbic acid or salt thereof, and dibutylhydroxytoluene. Among these examples, cysteine hydrochloride hydrate (typically cysteine hydrochloride monohydrate) is preferable from the physiological point of view.

The buffer agent may be selected from any existing ones used for injection solution. It includes, for example, phosphoric acid or a salt thereof or a hydrate thereof (e.g., sodium hydrogen phosphate hydrate), citric acid or a salt thereof or a hydrate thereof, and acetic acid or a salt thereof or a hydrate thereof. Of these examples, anhydrous sodium hydrogen phosphate or hydrate thereof is preferable from the standpoint of stability and solubility of the drug substance. Sodium hydrogen phosphate should preferably in the form of disodium salt. Sodium hydrogen phosphate hydrate should preferably in the form of dihydrate, heptahydrate, or dodecahydrate.

The acetaminophen aqueous solution may optionally contain one or two or more species of additional pharmaceutical active ingredients, such as analgesic, antiinflammatory drug, antiemetic, antiepileptic drug, antidepressant, and antiviral drug, or excipients, such as polyol, complexing agent, alkanol, and vitamin.
The acetaminophen aqueous solution may be prepared in any manner without specific restrictions so long as a homogenous solution is obtained without the acetaminophen and aforementioned ingredients undergoing deterioration and decomposition.

The process for preparing the acetaminophen aqueous solution may be carried out in the air or in an atmosphere of inert gas or in an atmosphere of atmosphere of air and inert gas.
In the case where the atmosphere is a mixture of air and inert gas, the mixing ratio (air to inert gas) by volume should be from 1:99 to 99:1, preferably from 10:90 to 90:10.

Moreover, the acetaminophen aqueous solution may undergo bubbling with an inert gas during and/or after its preparation so that the oxygen content therein is reduced. Alternatively, the acetaminophen aqueous solution may be filled as such in the oxygen-permeable container without bubbling with an inert gas during or after its preparation.
The inert gas to be used for bubbling into the acetaminophen aqueous solution may be any of nitrogen gas, argon gas, and carbon dioxide gas, with nitrogen gas being preferable on account of its availability at low prices.

The acetaminophen aqueous solution is subsequently filled in an oxygen-permeable container. The oxygen-permeable container is not specifically restricted so long as it is made of any material which is permeable to oxygen but impermeable to liquid (water), nontoxic, free of adsorption of acetaminophen, and resistant to heat for moist heat sterilization.
The oxygen-permeable container has an oxygen permeability not lower than 500 cm³/m²/day measured at 23 degree C and 90% RH under normal pressure (1013 hPa), more preferably not lower than 700 cm³/m²/day, and most desirably from 700 to 10000 cm³/m²/day.

So long as the oxygen-permeable container has an oxygen permeability not lower than 200 cm³/m²/day measured at 23 degree C and 90% RH under normal pressure (1013 hPa), it permits oxygen brought when filling in the oxygen-permeable container and contained in the acetaminophen aqueous solution and the head space of the container to be absorbed and removed sufficiently through the oxygen-permeable container by the oxygen scavenger.
The oxygen permeable container should preferably have as high oxygen permeability as possible so that it permits oxygen contained in the acetaminophen aqueous solution filled therein and the head space therein to be absorbed and removed easily by the oxygen scavenger.

The oxygen-permeable container may be either flexible one or solid one. It should preferably be formed from an organic polymer which is permeable to oxygen but impermeable to liquid (water). It should more preferably be in the form of film or sheet formed from an organic polymer which is permeable to oxygen but impermeable to liquid (water). It should most preferably be in the form of soft (flexible) film or sheet formed from an organic polymer which is permeable to oxygen but impermeable to liquid (water).

The organic polymer permeable to oxygen but impermeable to liquid (water) from which the oxygen-permeable container is formed is selected from polyethylene resin, polypropylene resin, a mixture of polyethylene resin and polypropylene resin, ethylene-vinyl acetate copolymer, polyvinyl chloride resin. An oxygen-permeable flexible container may be formed from an organic polymer composition in which a solid organic polymer as a base is incorporated with a plasticizer or a compatible soft organic polymer (such as thermoplastic elastomers including styrene-based thermoplastic elastomer and olefin-based thermoplastic elastomer).

The oxygen-permeable container may be of single-layer structure or multi-layer structure having two or more layers formed from the foregoing organic polymer.
The oxygen-permeable container, which is formed from oxygen-permeable organic polymer film or sheet, should have a wall thickness of 50 to 500 micrometers, preferably 100 to 300 micrometers, so that it meets requirements for oxygen permeability and container performance. A thickness equal to or larger than 50 micrometers is enough for the container to have sufficient strength and sufficient resistance to moist heat sterilization. Also, a thickness equal to or smaller than 500 micrometers is desirable for oxygen permeability required and also for the bag-type container for the acetaminophen injection solution preparation to have adequate flexibility.

The oxygen-permeable container should preferably have a port through which the acetaminophen aqueous solution (or injection solution) filled in the container is discharged from the container for administration to the patient. The port may be provided with an elastic stopper in the case where an injection needle is used for discharging. The elastic stopper, which is made of elastomer or rubber, preferably permits an injection needle to be pierced and the pierced hole is closed (for resealing) after the injection needle has been removed. The material of the elastic stopper includes butyl rubber, isoprene rubber, chlorinated butyl rubber (chloroprene), of the like. Moreover, the elastic stopper may have its surface (at least in contact with the acetaminophen aqueous solution) coated with a layer of fluorine-based resin, polyparaxylene, DLC (diamond-like carbon), polypropylene, polyethylene or the like, which is attached thereto or deposited thereon, so that any components contained in the elastic stopper does not dissolve in the acetaminophen aqueous solution.

The oxygen-permeable container may be formed in any manner without specific restrictions. Any adequate manufacturing process may be adopted according to the type of material used and the shape of the container. Examples of the manufacturing process include extrusion blow molding, injection blow molding, inflation molding, injection molding, casting, heat sealing of flat film or sheet to close the peripheries by folding, heat sealing of cylindrical film or sheet to close the top and bottom, and providing the cylindrical body with a bottom and cap.

The oxygen-permeable container is not specifically restricted in its size. An adequate size, which depends on the form and concentration of the acetaminophen injection solution preparation, is usually such that the internal volume is 20 to 500 cm³, preferably 50 to 100 cm³. This size is convenient for handling and patient's water control.

The filling of the acetaminophen aqueous solution in the oxygen-permeable container may be performed in the air, in an atmosphere of inert gas, or in an atmosphere of a mixture of air and inert gas. The inert gas may be nitrogen gas, argon gas, carbon dioxide gas, etc., for example. Nitrogen gas is preferable because of its availability at low prices. Alternatively, the filling step may be followed by deaeration.

In the case where the filling of the acetaminophen aqueous solution in the oxygen-permeable container is performed in an atmosphere of a mixture of air and inert gas, the mixing ratio of air to inert gas should preferably be from 1:99 to 99:1 (by volume), more preferably from 10:90 to 90:10 (by volume).

The oxygen-permeable container filled with the acetaminophen aqueous solution subsequently undergoes moist heat sterilization. This procedure may be accomplished by using steam as a sterilizing medium and air as a pressurizing medium, or using steam as a sterilizing medium and inert gas as a pressurizing medium, or using steam as a sterilizing medium and a mixture of air and inert gas as a pressurizing medium. The inert gas for this purpose may be nitrogen gas, argon gas, carbon dioxide gas, etc., for example. Nitrogen gas is preferable because of its availability at low prices.

In the case where a mixture of air and inert gas is used as a pressurizing medium, the mixing ratio of air to inert gas should preferably be from 1:99 to 99:1 (by volume), more preferably from 10:90 to 90:10 (by volume).
The moist heat sterilization may be accomplished under any conditions (temperature, pressure, time, etc.) normally employed for sterilization of injection solution preparations. For example, the temperature should be 100 to 129 degree C, preferably 115 to 124 degree C.

According to the present invention, at least one or two or all of the step for preparing the acetaminophen aqueous solution before filling in the container, the step for filling the acetaminophen aqueous solution in an oxygen-permeable container, and the step for moist heat sterilization may be performed in an atmosphere into which an inert gas is being introduced or in an atmosphere of an inert gas. Alternatively, all of the steps may be performed in an atmosphere of air. Combinations of such modes are illustrated below.
(1) Both of preparation of the acetaminophen aqueous solution in an inert gas atmosphere (realized in an inert gas booth), and bubbling of the acetaminophen aqueous solution with an inert gas are performed. The acetaminophen aqueous solution is filled in the oxygen-permeable container in an inert gas atmosphere, and the oxygen-permeable container holding the acetaminophen aqueous solution undergoes moist heat sterilization that employs an inert gas as a pressurizing medium.
(2) Either one or both of the preparation of the acetaminophen aqueous solution in an inert gas atmosphere (realized in an inert gas booth) and bubbling of the acetaminophen aqueous solution with an inert gas are performed. The acetaminophen aqueous solution is filled in the oxygen-permeable container in an inert gas atmosphere, and the oxygen-permeable container holding the acetaminophen aqueous solution undergoes moist heat sterilization that employs air as a pressurizing medium.
(3) Either one or both of the preparation of the acetaminophen aqueous solution in an inert gas atmosphere (realized in an inert gas booth) and bubbling of the acetaminophen aqueous solution with an inert gas are not performed. On the other hand, the acetaminophen aqueous solution is filled in the oxygen-permeable container in an inert gas atmosphere, and the oxygen-permeable container holding the acetaminophen aqueous solution undergoes moist heat sterilization that employs an inert gas as a pressurizing medium.
(4) Either one or both of the preparation of the acetaminophen aqueous solution in an inert gas atmosphere (realized in an inert gas booth) and bubbling of the acetaminophen aqueous solution with an inert gas are not performed. Moreover, the acetaminophen aqueous solution is filled in the oxygen-permeable container in an inert gas atmosphere, but the oxygen-permeable container holding the acetaminophen aqueous solution undergoes moist heat sterilization that employs air as a pressurizing medium.
(5) Either one or both of the preparation of the acetaminophen aqueous solution in an inert gas atmosphere (realized in an inert gas booth) and bubbling of the acetaminophen aqueous solution with an inert gas are performed. The acetaminophen aqueous solution is filled in the oxygen-permeable container in the air, and the oxygen-permeable container holding the acetaminophen aqueous solution undergoes moist heat sterilization that employs an inert gas as a pressurizing medium.
(6) The acetaminophen aqueous solution is prepared in the absence of an inert gas atmosphere (realized in an inert gas booth), and the acetaminophen aqueous solution does not undergo bubbling with an inert gas. The acetaminophen aqueous solution is filled in the oxygen-permeable container in the air, and the oxygen-permeable container holding the acetaminophen aqueous solution undergoes moist heat sterilization that employs air as a pressurizing medium.
(7) In any one of the modes (1) to (6) mentioned above, the acetaminophen aqueous solution is filled in the oxygen-permeable container in a mixture of air and inert gas, and/or the oxygen-permeable container holding the acetaminophen aqueous solution undergoes moist heat sterilization that employs a mixture of air and inert gas as a pressurizing medium.

After moist heat sterilization, the oxygen-permeable container holding the acetaminophen aqueous solution is placed and sealed, together with an oxygen scavenger, in an oxygen-impermeable outer container.
The oxygen-impermeable outer container (packing container) is not specifically restricted in its performance so long as it is capable of accommodating the oxygen-permeable container holding the acetaminophen aqueous solution, and oxygen scavenger and it is impermeable to oxygen and non-toxic.

The oxygen-impermeable outer container (packing container) has an oxygen permeability not higher than 1.0 cm³/m²/day measured at 23 degree C and 90% RH under normal pressure (1013 hPa). The oxygen permeability should preferably be not higher than 0.3 cm³/m²/day, and more preferably not higher than 0.1 cm³/m²/day.

So long as the oxygen-impermeable outer container (packing container) has an oxygen permeability not higher than 1.0 cm³/m²/day measured at 23 degree C and 90% RH under normal pressure (1013 hPa), it permits the oxygen scavenger therein to sufficiently absorb and remove oxygen released from the acetaminophen aqueous solution held in the oxygen-permeable container and the head space in the oxygen-permeable container through the oxygen-permeable container while shutting out air (oxygen) outside the oxygen-impermeable outer container.

The oxygen-impermeable outer container (packing container) should preferably have as low oxygen permeability as possible so that it permits the oxygen scavenger to easily absorb and remove oxygen released from the acetaminophen aqueous solution held in the oxygen-permeable container and the head space in the oxygen-permeable container.
The oxygen-impermeable outer container (packing container) may be either flexible one or solid one. The oxygen-impermeable outer container preferably is formed from an impermeable organic polymer film or sheet. The impermeable organic polymer film and sheet preferably have flexibility.

The oxygen-impermeable outer container (packing container) may be formed from an oxygen-impermeable metal foil (such as aluminum foil), a deposited film or sheet, an organic polymer film or sheet coated with DLC (diamond-like coating), an oxygen-impermeable film or sheet formed from an oxygen-impermeable resin such as polyvinyl alcohol or ethylene-vinyl alcohol, a laminated film or sheet, and a multiayer film or sheet. The deposited film or sheet may be composed of a substrate film or sheet made of organic polymer and a coating layer. The coating layer deposited on the substrate film or sheet may be ceramics (such as silicon oxide, aluminum oxide, and titanium oxide) or metal. The deposited film or sheet includes aluminum-deposited organic polymer film or sheet, silica-deposited organic polymer film or sheet, titanium oxide-deposited organic polymer film or sheet. The laminated film or sheet may be composed of the oxygen-impermeable resin layers such as polyvinyl alcohol or ethylene-vinyl alcohol, which are laminated one over another. The multi-layer film or sheet may be composed of one or two or more of the above-mentioned oxygen-impermeable film or sheet and a layer made of other organic polymer than mentioned-above, which are laminated one over another.

In the case where the oxygen-impermeable outer container (packing container) is formed from an oxygen-impermeable film or sheet of organic polymer, the inner layer of the oxygen-impermeable film or sheet should preferably be formed from a heat-sealable organic polymer because the oxygen-impermeable outer container (packing container) is usually formed by heat sealing the periphery of folded film or sheet.

The oxygen-impermeable outer container (packing container) may be formed from any materials exemplified below without specific restrictions.
Multi-layer film or sheet composed of polypropylene (PP), silica-deposited polyethylene terephthalate (PET), and polypropylene (PP), which are laminated one over another in the order mentioned
Multi-layer film or sheet composed of biaxially oriented polyamide (OPA), polyethylene (PE), aluminum-deposited PET, and polyethylene, which are laminated one over another in the order mentioned

Multi-layer film or sheet composed of OPA, PE, aluminum-deposited PET, and PE, which are laminated one over another in the order mentioned
Multi-layer film or sheet composed of OPA, PE, aluminum foil, PE, and PE, which are laminated one over another in the order mentioned
Multi-layer film or sheet composed of OPA, PE, aluminum foil, PE, PET, and PE, which are laminated one over another in the order mentioned

Multi-layer film or sheet composed of PET, PE, aluminum-deposited PET, PE, ethylene-vinyl acetate copolymer (EVA), and PE, which are laminated one over another in the order mentioned
Multi-layer film or sheet composed of polyvinylidene chloride, PE, aluminumdeposited PET, and PE, which are laminated one over another in the order mentioned Multi-layer film or sheet composed of PET, aluminum-deposited ethylene-vinyl alcohol copolymer (EVOH), and PE, which are laminated one over another in the order mentioned

In the case where the oxygen-impermeable outer container (packing container) is formed from an oxygen-impermeable film or sheet of organic polymer, the oxygen-impermeable film or sheet should have a thickness of 50 to 150 micrometers, preferably 65 to 100 micrometers, so that it meets requirements for oxygen impermeability as well as container performance. A thickness not smaller than 50 micrometers is enough for the oxygen-impermeable film or sheet to provide the outer container (packing container) with adequate strength. A thickness not larger than 150 micrometers is suitable for the outer container of flexible bag type for the acetaminophen injection solution preparation to have adequate flexibility and light weight.

The oxygen scavenger, which is packed in the oxygen-impermeable outer container (packing container) together with the oxygen-permeable container holding the acetaminophen aqueous solution, is not specifically restricted so long as it is non-toxic and capable of effectively absorbing oxygen to reduce oxygen concentration in the at-atmosphere.
Examples of the oxygen scavenger include metal powder (such as iron powder), inorganic substance (such as sulfite and metal halide), and organic substance (such as ascorbic acid and polyphenol) as a main material. Any one of them may be used so long as it is non-toxic and excellent in oxygen absorptivity.

Moreover, it is also possible to use a substance which absorbs oxygen and carbon dioxide or which absorbs oxygen and releases carbon dioxide.
The oxygen scavenger may come in various forms, such as package including an oxygen-permeable bag and an oxygen-scavenging substance contained therein, a film formed by kneading with an oxygen absorbing agent, a container molded with an oxygen-absorbing agent, and a layered object which including oxygen-impermeable outer layers and an oxygen-absorbing inner layer, such that the sides thereof absorb oxygen. Any one of them may be used so long as it is non-toxic and good in oxygen absorptivity.

Among known commercial products is Ageless (registered trade name)" (from Mitsubishi Gas Chemical Company, Inc.), which is suitable for the present invention. The oxygen-scavenger should be one which absorbs oxygen in an amount not less than 100 cm³, preferably not less than 200 cm³, and more preferably not less than 400 cm³, at normal temperature (25 degree C) and atmospheric pressure for 20 hours, from the acetaminophen aqueous solution in an amount of 100 mL, held in the oxygen-permeable container.

A preferred example of the oxygen scavenger is Ageless (registered trade name) ZH-200" (from Mitsubishi Gas Chemical Company, Inc.), which absorbs oxygen in an amount equal to or more than 200 cm³ per 20 hours at atmospheric pressure.
An example of the packaged acetaminophen injection solution preparation of this invention is shown in Fig. 1.

The packaged acetaminophen injection solution preparation 1 of this embodiment comprises a sealed oxygen-permeable container 3 filled with acetaminophen aqueous solution of pH 4 to 8 containing acetaminophen as an active ingredient and sterilized by moist heat sterilization, an oxygen scavenger 4 and an oxygen-impermeable packaging container 2 enclosed the sealed oxygen-permeable container 3 filled with the acetaminophen aqueous solution and the oxygen scavenger 4. The oxygen-impermeable packaging container 2 is sealed and the oxygen scavenger 4 is positioned in a space between the packaging container 2 and the sealed oxygen-permeable container 3 filled the acetaminophen aqueous solution.

The sealed oxygen-permeable container 3 comprise a container body 31 having an inner space for storing the acetaminophen aqueous solution and a port communicating to the space and a sealing member 32 for closing the port of the container body 31. The container body 31 is a flexible bag made of an oxygen-permeable sheet or film of organic polymer as described above. The sealing member 32 is desirable to be able to a discharge puncture needle.

The oxygen-impermeable packaging container 2 is a bag-shaped. The oxygen-impermeable packaging container 2, after storing the oxygen absorber 4 and the container body 3, the opening section of the oxygen-impermeable packaging container 2 is sealed by heat sealing. The oxygen-impermeable outer container is made of an oxygen-impermeable film or sheet of organic polymer as described above. As shown in Fig.1, the oxygen scavenger 4 may be fixed to an inner surface of the oxygen-impermeable packaging container 2.
The oxygen scavenger may be provided directly on the inner surface of the packaging container. Further, the oxygen scavenger may be provided directly on the outer surface of the sealed oxygen-permeable container.

### (Examples)

The present invention will be described in more detail with reference to the following examples, which are not intended to restrict the scope thereof.
In the examples, the following procedure was employed to measure the oxygen concentration in the water for injection and the acetaminophen aqueous solution. Measurement of oxygen concentration in the water for injection and the acetaminophen aqueous solution
The oxygen concentration in the water and solution was measured by using a dissolved oxygen measuring sensor Inlab 605 (from Mettler Toledo).

### Example 1

(1) An acetaminophen aqueous solution was prepared as follows. An adequate amount of water for injection, which had been warmed at 35 degree C, underwent nitrogen gas bubbling in an atmosphere of nitrogen gas (or in a nitrogen gas booth), so that the oxygen concentration therein was kept not higher than 1 mg/L. The water for injection was given 1925 g of D-mannitol, 13 g of disodium hydrogen phosphate dodecahydrate, and 500 g of acetaminophen, with stirring for dissolution. After cooling to room temperature, 12.5 g of L-cysteine hydrochloride monohydrate was added and dissolve therein. The resulting solution was adjusted to pH 5.5 with sodium hydroxide and hydrochloric acid and then added water for injection so that the total amount of the solution became 50 L.
(2) 100 mL each of the acetaminophen aqueous solution obtained in the step (1) above was filled in a flexible bag of oxygen-permeable multi-layer polypropylene film (100 mL in nominal capacity), followed by sealing with an isoprene rubber stopper. The multi-layer film is composed of three layers of polypropylene film, 264 micrometers in total thickness. The multi-layer film has an oxygen permeability of 809 cm³/m²/day at 23 degree C, 90% RH under normal pressure. The filling step was carried out in an atmosphere of a mixture of nitrogen gas and air (at a ratio of 80:20 by volume).
(3) After filling with the acetaminophen aqueous solution in the step (2) above, the oxygen-permeable flexible bag (or the oxygen-permeable multi-layer polypropylene flexible bag) underwent moist heat sterilization with nitrogen gas as a pressurizing medium (at 121 degree C for 15 minutes).
(4) After the moist heat sterilization in the step (3) above, the oxygen-permeable container filling the acetaminophen aqueous solution was placed in an oxygen-impermeable bag together with one piece of Ageless (registered trade name) ZH-200 (from Mitsubishi Gas Chemical Company, Inc.), which absorbs oxygen in an amount equal to or more than 200 cm³ per 20 hours at atmospheric pressure. The oxygen-impermeable bag is one formed from three-layer film of PP, silica-deposited PET, and PP, with its three sides sealed and one side open, which has a thickness of 66 micrometers and an oxygen permeability of 0.15 cm³/m²/day at 23 degree C, 90% RH under normal pressure. After the oxygen-permeable container and Ageless (registered trade name) ZH-200 have been placed in the oxygen-impermeable bag, the open side of the oxygen-impermeable bag was sealed by heat sealing. In this way there was obtained the packaged acetaminophen injection solution preparation as desired.

### Example 2

(1) The acetaminophen aqueous solution was prepared in the same way as in the step (1) in Example 1 above, except that nitrogen gas bubbling was not performed and the entire step was carried out in the air without using the nitrogen booth.
(2) 100 mL each of the acetaminophen aqueous solution obtained in the step (1) above was filled in the same oxygen-permeable container as used in the step (2) in Example 1 (which is an oxygen-permeable multi-layer polypropylene flexible bag, 100 mL in nominal capacity) in the air. After filling, the flexible bag was sealed with an isoprene rubber stopper.
(3) After filling with the acetaminophen aqueous solution in the step (2) above, the oxygen-permeable flexible bag (or the oxygen-permeable multi-layer polypropylene flexible bag) underwent moist heat sterilization in the same way as in the step (3) in Example 1.
(4) After the moist heat sterilization in the step (3) above, the oxygen-permeable container holding the acetaminophen aqueous solution was placed in the same oxygen-impermeable bag (with its three sides sealed) as used in the step (4) in Example 1, together with one piece of the same oxygen scavenger as used in the step (4) in Example 1. After the oxygen-permeable container and the oxygen scavenger have been placed in the oxygen-impermeable bag, the open side of the oxygen-impermeable bag was sealed by heat sealing. In this way there was obtained the packaged acetaminophen injection solution preparation as desired.

### Example 3

(1) The acetaminophen aqueous solution was prepared in the same way as in the step (1) in Example 1 above, except that nitrogen gas bubbling was not performed and the entire step was carried out in the air without using the nitrogen booth.
(2) 100 mL each of the acetaminophen aqueous solution obtained in the step (1) above was filled in the same oxygen-permeable container as used in the step (2) in Example 1 (which is an oxygen-permeable multi-layer polypropylene flexible bag, 100 mL in nominal capacity) in the air. After filling, the flexible bag was sealed with an isoprene rubber stopper.
(3) After filling with the acetaminophen aqueous solution in the step (2) above, the oxygen-permeable flexible bag (or the oxygen-permeable multi-layer polypropylene flexible bag) underwent moist heat sterilization in the same way as in the step (3) in Example 1, except that air was used as the pressurizing medium.
(4) After the moist heat sterilization in the step (3) above, the oxygen-permeable container holding the acetaminophen aqueous solution was placed in the same oxygen-impermeable bag (with its three sides sealed) as used in the step (4) in Example 1, together with one piece of the same oxygen scavenger as used in the step (4) in Example 1. After the oxygen-permeable container and the oxygen scavenger have been placed in the oxygen-impermeable bag, the open side of the oxygen-impermeable bag was sealed by heat sealing. In this way there was obtained the packaged acetaminophen injection solution preparation as desired.

### Example 4

(1) The acetaminophen aqueous solution was prepared and nitrogen gas bubbling was performed in the same way as in the step (1) in Example 1 above, except that the entire step was carried out in the air without using the nitrogen booth.
(2) 100 mL each of the acetaminophen aqueous solution obtained in the step (1) above was filled in the same oxygen-permeable container as used in the step (2) in Example 1 (which is an oxygen-permeable multi-layer polypropylene flexible bag, 100 mL in nominal capacity) in the air. After filling, the flexible bag was sealed with an isoprene rubber stopper.
(3) After filling with the acetaminophen aqueous solution in the step (2) above, the oxygen-permeable flexible bag (or the oxygen-permeable multi-layer polypropylene flexible bag) underwent moist heat sterilization in the same way as in the step (3) in Example 1.
(4) After the moist heat sterilization in the step (3) above, the oxygen-permeable container holding the acetaminophen aqueous solution was placed in the same oxygen-impermeable bag (with its three sides sealed) as used in the step (4) in Example 1, together with one piece of the same oxygen scavenger as used in the step (4) in Example 1. After the oxygen-permeable container and the oxygen scavenger have been placed in the oxygen-impermeable bag, the open side of the oxygen-impermeable bag was sealed by heat sealing. In this way there was obtained the packaged acetaminophen injection solution preparation as desired.

### Comparative Example 1

(1) The steps (1) to (3) in Example 1 were repeated to obtain the high-pressure steam-sterilized oxygen-permeable container (or oxygen-permeable multi-layer polypropylene flexible bag) holding the acetaminophen aqueous solution.
(2) After the moist heat sterilization in the step (1) above, the oxygen-permeable container (or oxygen-permeable multi-layer polypropylene flexible bag) holding the acetaminophen aqueous solution was placed in the same oxygen-impermeable bag (with its three sides sealed) as used in the step (4) in Example 1, without the oxygen scavenger. After the oxygen-permeable container has been placed in the oxygen-impermeable bag, the open side of the oxygen-impermeable bag was sealed by heat sealing. In this way there was obtained the packaged acetaminophen injection solution preparation as desired.

### Comparative Example 2

(1) The step (1) in Example 1 was repeated to obtain the acetaminophen aqueous solution, except that the process was carried out in the air without using the nitrogen booth and the nitrogen gas bubbling was not performed.
(2) 100 mL each of the acetaminophen aqueous solution obtained in the step (1) above was filled in the same oxygen-permeable container as used the step (2) in Example 1 (which is an oxygen-permeable multi-layer polypropylene flexible bag, 100 mL in nominal capacity) in the air. After filling, the flexible bag was sealed with an isoprene rubber stopper.
(3) The step (3) in Example 1 was repeated to perform moist heat sterilization on the oxygen-permeable container (or the oxygen-permeable multi-layer polypropylene flexible bag) holing the acetaminophen aqueous solution obtained in the step (2) above, except that air was used as the pressurizing medium.
(4) After the moist heat sterilization in the step (3) above, the oxygen-permeable container holding the acetaminophen aqueous solution was placed in the same oxygen-impermeable bag (with its three sides sealed) as used in the step (4) in Example 1, without the oxygen scavenger. After the oxygen-permeable container has been placed in the oxygen-impermeable bag, the open side of the oxygen-impermeable bag was sealed by heat sealing. In this way there was obtained the packaged acetaminophen injection solution preparation as desired.

The packaged acetaminophen injection solution preparations obtained in Examples 1 to 4 and Comparative Examples 1 and 2 were found to have the composition and pH value as shown in Table 1.

### [Table 1]

**Table 1**

| Component | Content in 100 mL of acetaminophen aqueous solution | Remarks |
|---|---|---|
| Acetaminophen | 1000 mg | |
| D-mannitol | 3850 mg | Tonicity agent |
| L-cysteine hydrochloride hydrate | 25 mg | Antioxidant |
| Sodium hydrogen phosphate hydrate | 26 mg | Buffer agent |
| Sodium hydroxide | q.s. | pH adjustor |
| Hydrochloric acid | q.s. | pH adjustor |
| Water for injection | q.s. | |
| pH | ---- | 5.5 |

Test 1 for measurement of oxygen concentration in the acetaminophen aqueous solution after moist heat sterilization and before filling in the outer container (packing container):
This test was performed to measure the oxygen concentration in the acetaminophen aqueous solutions each obtained in the step (3) in Example 1, in the step (3) in Example 2, in the step (3) in Example 3, in the step (3) in Example 4, in the step (1) in Comparative Example 1, and in the step (3) in Comparative Example 2. All the samples were collected after moist heat sterilization and before filling in the outer container (packing container). The results of measurements are shown in Table 2 through 4.

Test 2 for measurement of oxygen concentration in the acetaminophen injection solution preparation:
This test was performed to measure the oxygen concentration in the acetaminophen injection solution preparations each obtained in the step (4) in Example 1, in the step (4) in Example 2, in the step (4) in Example 3, in the step (4) in Example 4, in the step (2) in Comparative Example 1, and in the step (4) in Comparative Example 2. All the samples are finished products. Measurements for the acetaminophen aqueous solution were carried out after storage in the air at room temperature for 24 hours and four days. The results of measurements are shown in Table 2 through 4.

### Test 3 for stability:

Each sample of the acetaminophen injection solution preparations obtained in Examples 1 to 4 and Comparative Examples 1 and 2 was tested for appearance, pH, content of L-cysteine hydrochloride hydrate, total amount of decomposition products, and residual ratio of acetaminophen. This test was performed immediately after production and after storage at 60 degree C for seven days and 21 days. The rest method and result are shown in Table 2 through 4 below.

### Test 4 for stability:

Each sample of the acetaminophen injection solution preparations obtained in Example 1 was tested for appearance, pH, content of L-cysteine hydrochloride hydrate, total amount of decomposition products, and residual ratio of acetaminophen. This test was performed after storage at 40 degree C for three months and six months. The result is shown in Table 2 through 4 below.
(1) Evaluation of appearance of acetaminophen aqueous solution in acetaminophen injection solution preparation:
   Each sample was examined for clarity and color.
(2) pH of acetaminophen aqueous solution in acetaminophen injection solution preparation:
   Each sample was tested for pH by using a pH meter.
(3) Content of L-cysteine hydrochloride hydrate in acetaminophen aqueous solution of acetaminophen injection solution preparation:
   Each sample was diluted with water to give a dilute solution of prescribed concentration. The dilute solution was examined for the content of L-cysteine hydrochloride hydrate (in terms of mg/100 mL) by liquid chromatography.
(4) Total amount of decomposition products in acetaminophen aqueous solution of acetaminophen injection solution preparation:
   The total amount (%) of individual decomposition products was obtained by liquid chromatography (in terms of areal percentage of peak areas of chromatograms).
(5) Residual ratio of acetaminophen in acetaminophen aqueous solution of acetaminophen injection solution preparation:
   Each sample was diluted with water to give a dilute solution of prescribed concentration. The dilute solution was examined for the content of acetaminophen by liquid chromatography. The residual ratio is expressed in terms of percentage (%) of the initial content (100%) before the test.

### [Table 2]

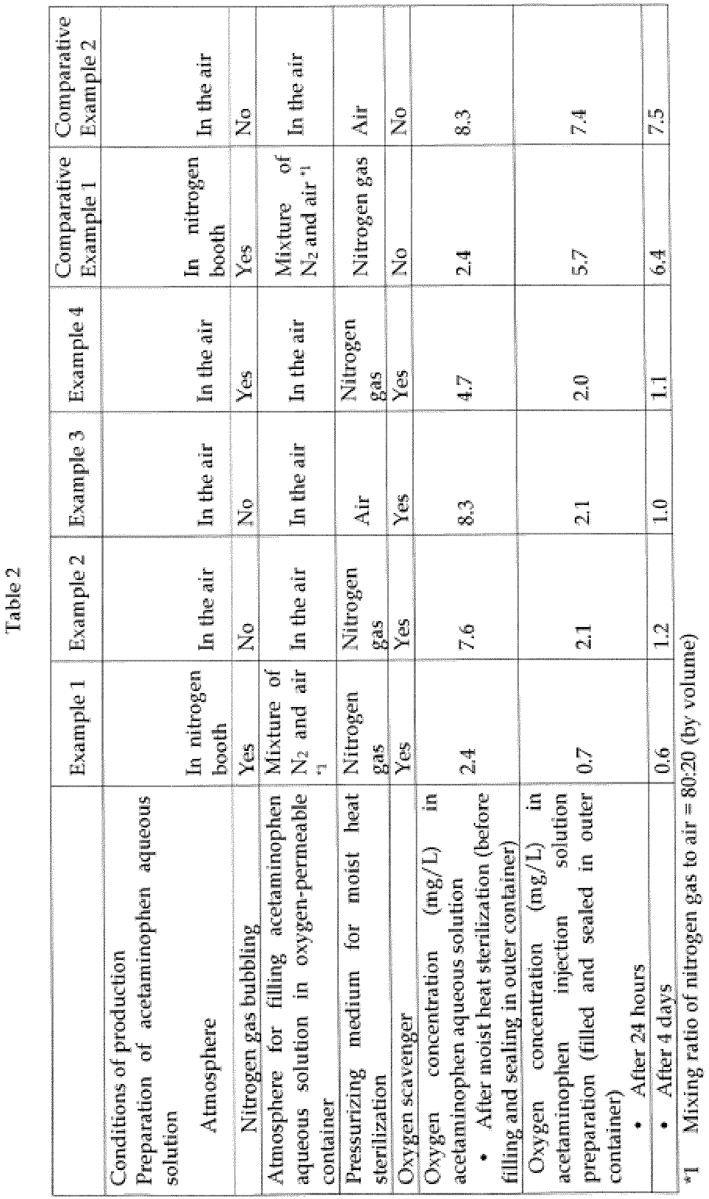

### [Table 3]

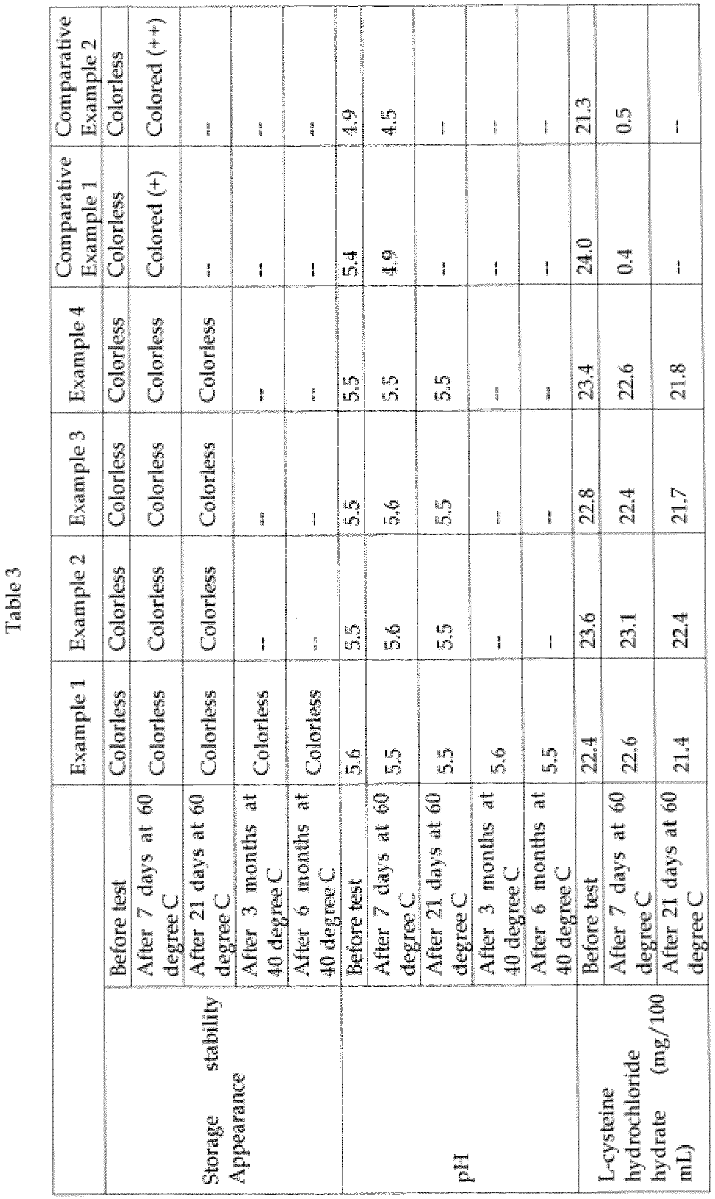

### [Table 4]

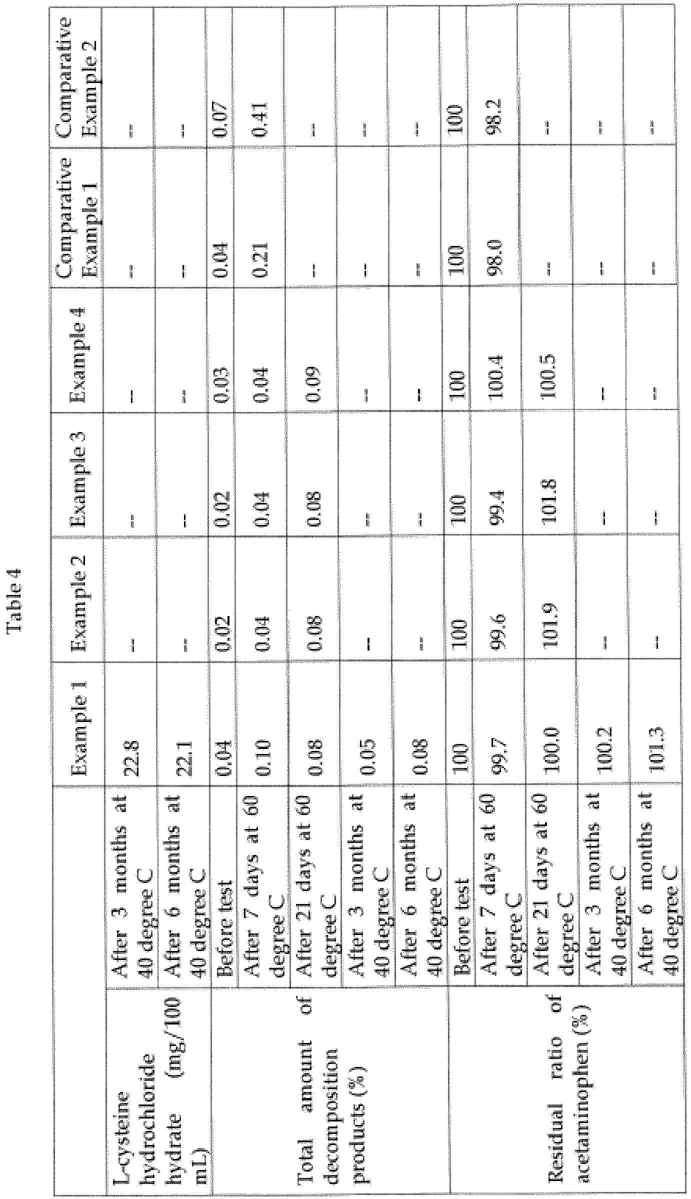

It is noted from Table 2 through 4 that the acetaminophen injection solution preparations according to Examples 1 to 4 are extremely superior in long-term storage stability.
By contrast, the acetaminophen injection solution preparations according to Comparative Examples 1 and 2 are inferior in long-term storage stability.

The acetaminophen injection solution preparation according to the present invention excels in long-term storage stability at room temperature although it takes on the liquid form. It keeps acetaminophen with very little deterioration and decomposition or and it retains acetaminophen in a high residual ratio even after storage for a long period of time. Consequently it can be effectively used for medical treatment, such as the curing of postoperative pain.

### Industrial Applicability

The acetaminophen injection solution preparation of the present invention is constructed as described below.
(1) A packaged acetaminophen injection solution preparation comprising:
   an oxygen-impermeable packaging container, and
   an acetaminophen injection solution preparation including a sealed oxygen-permeable container and an acetaminophen aqueous solution of pH 4 to 8 containing acetaminophen as an active ingredient filled in said sealed oxygen-permeable container, and
   an oxygen scavenger.
   wherein:
      - said acetaminophen injection solution preparation is a moist heat-sterilized acetaminophen injection solution preparation,
      - said oxygen-impermeable packaging container encloses said moist heat-sterilized acetaminophen injection solution preparation and said oxygen scavenger, and is sealed,
      - said oxygen-nermeable container has an oxygen permeability not lower than 500 cm³m²/day measured at 23 degree C and 90% RH under normal pressure (1013 hPa),
         and
      - said oxygen scavenger is positioned in a space between said sealed oxygen-impermeable container and said moist heat-sterilized acetaminophen injection solution preparation to absorb oxygen within said sealed oxygen-impermeable packaging container.

The packaged acetaminophen injection solution preparation according to the present invention is composed of an acetaminophen aqueous solution, an oxygen-permeable inner container filling it, and an oxygen-impermeable outer container (packing container) storing together the inner container and the oxygen scavenger. The filled inner container undergoes moist heat sterilization and then it and an oxygen scavenger are placed in the outer container (packing container) and then the outer container (packing container) is sealed. Unlike the existing acetaminophen injection solution preparation using a glass vial, the present invention does not need stringent oxygen control by inert gas displacement at the time of preparation of acetaminophen aqueous solution, at the time of filling acetaminophen aqueous solution into a container, and at the subsequent time of moist heat sterilization. Hence the present invention permits at least one or all of the steps for preparing the acetaminophen aqueous solution before filling in a container, filling the acetaminophen aqueous solution in a container, and moist heat sterilization to be carried out in the air. Moreover, it does not need such a strict control as practiced in the past even when an inert gas is employed for deoxygenation. Despite the simplified oxygen control process, the acetaminophen injection solution preparation excels in long-term storage stability, allowing acetaminophen therein to remain intact at an extremely high ratio even in the case of storage at room temperature for three years or longer.

If the oxygen-permeable container is made of an oxygen-permeable film or sheet of organic polymer and the oxygen-impermeable packaging container is made of an oxygen-impermeable film or sheet of organic polymer, it is easy to transport and handle without damage by dropping or tipping.

The packaged acetaminophen injection solution preparation according to the present invention excels in long-term storage stability unlike the existing one which is inevitably subject to oxygen contamination despite strict oxygen control in the individual steps for preparing the acetaminophen aqueous solution, filling it in an oxygen-impermeable outer container (packing container), and performing moist heat sterilization in an inert gas atmosphere.

## Claims

1. A packaged acetaminophen injection solution preparation comprising:
an oxygen-impermeable packaging container, and
an acetaminophen injection solution preparation including a sealed oxygen-permeable container and an acetaminophen aqueous solution of pH 4 to 8 containing acetaminophen as an active ingredient filled in said sealed oxygen-permeable container, and
an oxygen scavenger,
wherein:
- said acetaminophen injection solution preparation is a moist heat-sterilized acetaminophen injection solution preparation,
- said oxygen-impermeable packaging container encloses said moist heat-sterilized acetaminophen injection solution preparation and said oxygen scavenger, and is sealed,
- said sealed oxygen-permeable container is a flexible container made of an oxygen-permeable
film or sheet of organic polymer selected from polyethylene resin, polypropylene resin, a mixture of polyethylene resin and polypropylene resin, ethylene-vinyl acetate copolymer, polyvinyl chloride resin, or of an organic polymer composition in which a solid organic polymer as a base is incorporated with a plasticizer or a compatible soft organic polymer, and
- said oxygen-permeable container has an oxygen permeability not lower than 500 cm³/m²/day measured at 23 degree C and 90% RH under normal pressure (1013 hPa),
- said oxygen-impermeable packaging container has an oxygen permeability not higher than 1.0 cm³/m²/day measured at 23 degree C and 90% RH under normal pressure (1013 hPa),
and
- said oxygen scavenger is positioned in a space between said sealed oxygen-impermeable container and said moist heat-sterilized acetaminophen injection solution preparation to absorb oxygen within said sealed oxygen-impermeable packaging container.

2. A packaged acetaminophen injection solution preparation according to claim 1, wherein said acetaminophen aqueous solution contains acetaminophen in an amount of 2 to 50 mg/mL.

3. A packaged acetaminophen injection solution preparation according to claim 1 or 2, wherein said acetaminophen aqueous solution contains at least one species of pH adjustor, tonicity agent, antioxidant, and buffer agent.

4. A packaged acetaminophen injection solution preparation according to claim 3 wherein said pH adjustor is sodium hydroxide and/or hydrochloric acid.

5. A packaged acetaminophen injection solution preparation according to claim 3 or 4, wherein said tonicity agent is mannitol.

6. A packaged acetaminophen injection solution preparation according to claim 3, wherein said antioxidant is cysteine hydrochloride hydrate.

7. A packaged acetaminophen injection solution preparation according to claim 3, wherein said buffer agent is disodium hydrogen phosphate hydrate.

## Patentansprüche

1. Verpacktes Acetaminophen-Injektionslösungspräparat, umfassend:
einen sauerstoffundurchlässigen Verpackungsbehälter, und
ein Acetaminophen-Injektionslösungspräparat, das einen versiegelten sauerstoffdurchlässigen Behälter und eine wässrige Acetaminophen-Lösung mit einem pH-Wert von 4 bis 8 enthält, die Acetaminophen als in den versiegelten sauerstoffdurchlässigen Behälter gefüllten Wirkstoff enthält, und
einen Sauerstofffänger,
wobei:
- das Acetaminophen-Injektionslösungspräparat ein in feuchter Hitze sterilisiertes Acetaminophen-Injektionslösungspräparat ist,
- der sauerstoffundurchlässige Verpackungsbehälter das in feuchter Hitze sterilisierte Acetaminophen-Injektionslösungspräparat und den Sauerstofffänger umschließt und versiegelt ist,
- der versiegelte sauerstoffdurchlässige Behälter ein flexibler Behälter aus einem sauerstoffdurchlässigen Film oder einer sauerstoffdurchlässigen Folie aus organischem Polymer ist, das aus Polyethylenharz, Polypropylenharz, einer Mischung von Polyethylenharz und Polypropylenharz, Ethylen-Vinylacetat-Copolymer, Polyvinylchloridharz oder aus einer organischen Polymerzusammensetzung ausgewählt ist, in der ein festes organisches Polymer als Basis mit einem Weichmacher oder einem kompatiblen weichen organischen Polymer enthalten ist, und
- der sauerstoffdurchlässige Behälter eine Sauerstoffdurchlässigkeit nicht unter 500 cm³/m²/Tag bei 23°C und 90% relativer Feuchte unter Normaldruck (1013 hPa) hat,
- der sauerstoffundurchlässige Verpackungsbehälter eine Sauerstoffdurchlässigkeit nicht höher als 1,0 cm³/m²/Tag bei 23°C und 90% relativer Feuchte unter Normaldruck (1013 hPa) hat,
und
- der Sauerstofffänger in einem Raum zwischen dem versiegelten sauerstoffundurchlässigen Behälter und dem in feuchter Hitze sterilisierten Acetaminophen-Injektionslösungspräparat positioniert ist, um Sauerstoff in dem versiegelten sauerstoffundurchlässigen Verpackungsbehälter zu absorbieren.

2. Verpacktes Acetaminophen-Injektionslösungspräparat nach Anspruch 1, wobei die wässrige Acetaminophenlösung Acetaminophen in einer Menge von 2 bis 50 mg/ml enthält.

3. Verpacktes Acetaminophen-Injektionslösungspräparat nach Anspruch 1 oder 2, wobei die wässrige Acetaminophenlösung mindestens eine Spezies eines pH-Einstellmittels, Tonizitätsmittels, Antioxidans und Puffermittels enthält.

4. Verpacktes Acetaminophen-Injektionslösungspräparat nach Anspruch 3, wobei das pH-Einstellmittel Natriumhydroxid und/oder Salzsäure ist.

5. Verpacktes Acetaminophen-Injektionslösungspräparat nach Anspruch 3 oder 4, wobei das Tonizitätsmittel Mannitol ist.

6. Verpacktes Acetaminophen-Injektionslösungspräparat nach Anspruch 3, wobei das Antioxidans Cysteinhydrochloridhydrat ist.

7. Verpacktes Acetaminophen-Injektionslösungspräparat nach Anspruch 3, wobei das Puffermittel Dinatriumhydrogenphosphathydrat ist.

## Revendications

1. Préparation de solution pour injection d'acétaminophène conditionnée comprenant :
un récipient de conditionnement imperméable à l'oxygène, et
une préparation de solution pour injection d'acétaminophène comportant un récipient perméable à l'oxygène scellé et une solution aqueuse d'acétaminophène de pH 4 à 8 contenant de l'acétaminophène en tant que principe actif introduit dans ledit récipient perméable à l'oxygène scellé, et
un désoxygénant,
dans laquelle :
- ladite préparation de solution pour injection d'acétaminophène est une préparation de solution pour injection d'acétaminophène stérilisée à la chaleur humide,
- ledit récipient de conditionnement imperméable à l'oxygène renferme ladite préparation de solution pour injection d'acétaminophène stérilisée à la chaleur humide et ledit désoxygénant, et est scellé,
- ledit récipient perméable à l'oxygène scellé est un récipient souple formé d'un film ou une feuille perméable à l'oxygène en polymère organique sélectionné parmi une résine de polyéthylène, une résine de polypropylène, un mélange de résine de polyéthylène et de résine de polypropylène, un copolymère d'éthylène-acétate de vinyle, une résine de chlorure de polyvinyle, ou d'une composition de polymère organique dans laquelle dans un polymère organique solide en tant que base est incorporé un plastifiant ou un polymère organique mou compatible,
et
- ledit récipient perméable à l'oxygène a une perméabilité à l'oxygène pas inférieure à 500 cm³/m²/jour mesurée à 23 °C et 90 % de HR dans des conditions de pression normales (1013 hPa),
- ledit récipient de conditionnement imperméable à l'oxygène a une perméabilité à l'oxygène pas supérieure à 1,0 cm³/m²/jour mesurée à 23 °C et 90 % de HR dans des conditions de pression normales (1013 hPa), et
- ledit désoxygénant est positionné dans un espace entre ledit récipient imperméable à l'oxygène scellé et ladite préparation de solution pour injection d'acétaminophène stérilisée à la chaleur humide pour absorber l'oxygène à l'intérieur dudit récipient de conditionnement imperméable à l'oxygène scellé.

2. Préparation de solution pour injection d'acétaminophène conditionnée selon la revendication 1, dans laquelle ladite solution aqueuse d'acétaminophène contient de l'acétaminophène en une quantité de 2 à 50 mg/ml.

3. Préparation de solution pour injection d'acétaminophène conditionnée selon la revendication 1 ou 2, dans laquelle ladite solution d'acétaminophène contient au moins une espèce d'ajusteur du pH, d'agent de tonicité, d'antioxydant, et d'agent tampon.

4. Préparation de solution pour injection d'acétaminophène conditionnée selon la revendication 3 dans laquelle ledit ajusteur du pH est l'hydroxyde de sodium et/ou l'acide chlorhydrique.

5. Préparation de solution pour injection d'acétaminophène conditionnée selon la revendication 3 ou 4, dans laquelle ledit agent de tonicité est le mannitol.

6. Préparation de solution pour injection d'acétaminophène conditionnée selon la revendication 3, dans laquelle ledit antioxydant est le chlorhydrate de cystéine hydraté.

7. Préparation de solution pour injection d'acétaminophène conditionnée selon la revendication 3, dans laquelle ledit agent tampon est l'hydrogénophosphate disodique hydraté.
